Europäisches Patentamt

European Patent Office

Office européen des brevets

(11) Veröffentlichungsnummer: **0 069 355**
A2

(12) # EUROPÄISCHE PATENTANMELDUNG

(21) Anmeldenummer: 82105885.6

(22) Anmeldetag: 01.07.82

(51) Int. Cl.³: **G 01 M 11/02**
G 01 M 11/08, G 01 N 3/08
G 01 B 11/10

(30) Priorität: 03.07.81 DE 3126356

(43) Veröffentlichungstag der Anmeldung:
12.01.83 Patentblatt 83/2

(84) Benannte Vertragsstaaten:
FR GB IT

(71) Anmelder: SIEMENS AKTIENGESELLSCHAFT Berlin
und München
Postfach 22 02 61
D-8000 München 22(DE)

(72) Erfinder: Douklias, Nikolaos, Dr.rer.nat.
Margeritenweg 17
D-8011 Kirchheim(DE)

(54) **Verfahren zum Prüfen von Objekten.**

(57) Es wird ein Verfahren zum Prüfen von Lichtleitfasern
oder Vorformen, aus denen die Lichtleitfasern hergestellt
werden, auf Fehlstellen beschrieben, bei dem durch Beleuchten der vorzugsweise frisch gezogenen und völlig unbehandelten Faser mit einem kohärenten Lichtstrahl ein Raumfrequenzspektrum des beleuchteten Teils erzeugt wird, das mit
einem auf einem Raumfrequenzfilter vorhandenen Raumfrequenzspektrum einer keine nennenswerten Fehlstellen aufweisenden Faser zur Deckung gebracht wird. Das Raumfrequenzfilter enthält das Raumfrequenzspektrum als Negativ.
Das durch das Filter hindurchgegangene Licht wird beispielsweise durch Rücktransformation ausgewertet. Es lassen sich
mit diesem Verfahren Fehlstellen sowie Brechungsindex-
und Dickenschwankungen nach Lage, Art und Größe erkennen. Daraus lassen sich beispielsweise zuverlässige Schlüsse über die Zerreißfestigkeit der Faser gewinnen und es
lassen sich deshalb durch das Verfahren Fasern zerstörungsfrei auf mechanische und optische Qualität überprüfen.

EP 0 069 355 A2

Croydon Printing Company Ltd.

SIEMENS AKTIENGESELLSCHAFT       Unser Zeichen
Berlin und München               VPA 81 P 7 0 6 9 E

## Verfahren zum Prüfen von Objekten

Die vorliegende Erfindung bezieht sich auf ein Verfahren zum Prüfen von Objekten nach dem Oberbegriff des Patentanspruchs 1.

Wie dort bereits angegeben, kommen als Objekte insbesondere Lichtleitfasern oder Vorformen, aus denen Lichtleitfasern ziehbar sind, in Frage.

Lichtleitfasern für die optische Nachrichtenübertragung müssen mit guten optischen Eigenschaften, wie z.B. niedrige Dämpfung, große numerische Apertur und geringe Impulsverbreiterung auch eine ausreichend hohe Zugfestigkeit aufweisen. Zum Schutz vor mechanischer und chemischer Beschädigung der Faseroberfläche werden die Fasern deshalb direkt während des Ziehprozesses beschichtet, vorwiegend mit Kunststoffen. Häufig weisen jedoch auch beschichtete Fasern eine nur geringe Festigkeit auf, weil beispielsweise die Faseroberfläche noch im Ofenraum von Ofenteilchen beschädigt wird oder bereits in der Vorform, aus der die Faser gezogen wird, Fremdteilchen und andere Störstellen vorhanden sind. Die Festigkeit einer Faser hängt von der Häufigkeit und der Größe derartiger, statistisch über die gesamte Faserlänge verteilter Fehlstellen ab.

Die Faserfestigkeit wird derzeit dadurch geprüft, daß lange Fasern, in der Regel von über einem Kilometer Länge, gewöhnlich in 20 Meter lange Faserstücke geteilt und in einer Zerreiß-Prüfvorrichtung bis zum Bruch belastet werden. Aus der statistischen Auswertung der Zerreißkräfte der einzelnen Proben, nach der sog. Weibull- Methode können dann Aussagen über die erreichte und die bei anderen Fasern zu erwartenden Festigkeiten gewonnen werden.

Häufig werden die Fasern nach der Beschichtung einem sog. Screen-Test unterzogen. Dabei wird die Faser über Rollen geführt und über die Gesamtlänge mit einer minimalen Zugkraft belastet, bei der die Faser nicht abreißen darf. Der mechanische Kontakt der Faser mit den Rollen unter Zugspannung kann zur Beschädigung der Beschichtung und der Faseroberfläche und damit zur Herabsetzung der Faserfestigkeit führen.

Zur Prüfung von Vorformen, aus denen Lichtleitfasern ziehbar sind, in der Regel Lichtleitstäbe, werden zur Zeit aufwendige oder ungenaue Verfahren angewandt, siehe dazu beispielsweise H.M. Presby and. D. Marcuse: Optical Fiber Preform Diagnostics, Appl. Opt. Band 18, Nr. 8 (1979).

Die Aufgabe der vorliegenden Erfindung besteht darin, ein Verfahren der eingangs genannten Art zu schaffen, mit welchem die Objekte zerstörungsfrei und zuverlässig auf Solleigenschaften hin überprüft werden können. Insbesondere sollen Lichtleitfasern zerstörungsfrei und zuverlässig auf ihre Zugfestigkeit hin überprüft werden können.

Diese Aufgabe wird durch die im kennzeichnenden Teil des Patentanspruchs 1 angegebenen Merkmale gelöst.

Bei dieser Lösung ist im Zusammenhang mit Lichtleitfasern von der Erkenntnis ausgegangen worden, daß die Festigkeit einer Faser von der Häufigkeit und der Größe der statistisch über die gesamte Faserlänge verteilten Fehlstellen abhängt und daß die Kenntnis über den Ort und die Größe der Fehlstellen genügt, um Aussagen über die Faserfestigkeit zu gewinnen, ohne daß Probestücke nach den bekannten Methoden zerrissen werden müssen. Zu ähnlichen Aussagen führt auch das Orten von Fehlstellen in der Vorform, aus der eine Lichtleitfaser gezogen wird.

Die angegebene Lösung weist im Zusammenhang mit Lichtleitfasern den Vorteil auf, daß alle Fehlstellen nach Lage, Größe und Art erfaßt werden, so daß zuverlässige Angaben über die Zerreißfestigkeit der Faser gemacht werden können. Dabei arbeitet dieses Verfahren zerstörungsfrei.

Im Gegensatz dazu werden bei bekannten Verfahren, bei denen die Fasern zerrissen werden müssen, nicht alle Fehlstellen der Faser erfaßt, sondern nur die, die in dem Probestück zur geringsten Zerreißfestigkeit führen. Fehlstellen, welche die Zerreißfestigkeit der Faser zwar herabsetzen, die aber nicht zur minimalen Zerreißfestigkeit, sondern zu einer höheren führen, werden bei diesen bekannten Verfahren nicht mit erfaßt.

Das vorgeschlagene Verfahren läßt sich aber nicht nur zum Prüfen von Lichtleitfasern oder Vorformen verwenden, sondern kann generell zum zerstörungsfreien Prüfen von körperlichen Objekten auf Solleigenschaften verwendet werden. Beispielsweise können körperliche Gegenstände hinsichtlich ihrer Form oder Größe auf ihre Übereinstimmung mit einer vorgegebenen Sollform oder Größe hin überprüft werden.

Bei dem vorgeschlagenen Verfahren kann so vorgegangen werden, wie es im Anspruch 2 angegeben ist.

Das Raumfrequenzfilter kann beispielsweise fotografisch hergestellt werden (Anspruch 3), jedoch ist oftmals

-4-   VPA  81 P 7 0 6 9 E

seine Herstellung mit Hilfe eines Rechners undPlotters (Anspruch 4) vorteilhafter, weil dabei starke Intensitätsunterschiede, die bei fotografischen Aufnahmen zu Problemen führen können, ausgeglichen werden können.

Bei Objekten, bei denen Fehler in gewisser Weise geordnet sind, kann vielfach ein Verfahren verwendet werden, wie es im Anspruch 5 angegeben ist.

Insbesondere kann dieses Verfahren bei transparenten Fasern, insbesondere Glasfasern für die optische Nachrichtentechnik oder stabförmigen Vorformen für solche Fasern, angewendet werden, wobei wegen der Form dieser Objekte und der in Form von Längsriefen in Erscheinung tretenden geordneten Fehler, die im Anspruch 6 angegebenen Maßnahmen ausreichen.

Zweckmäßigerweise wird so ausgewertet, wie es im Anspruch 7 angegeben ist.

Vorzugsweise wird dabei so vorgegangen, wie es im Anspruch 8 angegeben ist.

Zweckmäßig ist es, das durch das Raumfrequenzfilter hindurchgegangene Licht oder das rekonstruierte Bild visuell über eine Fernsehkamera oder mittels eines Detektorfeldes und einem Kurvenschreiber oder Rechner auszuwerten (Anspruch 9).

Es kann auch so ausgewertet werden, wie es im Anspruch 10 angegeben ist.

Das vorgeschlagene Verfahren kann vorteilhaft zum Prüfen von bewegten, insbesondere faser- oder stabförmigen Objekten verwendet werden.

Eine vorteilhafte Vorrichtung zur Durchführung eines vorgeschlagenen Verfahrens ist gekennzeichnet durch eine einen kohärenten Lichtstrahl erzeugende Lichtquelle und durch eine auf der Strahlachse angeordnete Transformationslinse, zwischen der und der Lichtquelle das zu prüfende Objekt im Strahl anzuordnen ist und in einer deren Brennebenen das Raumfrequenzfilter anzuordnen ist (Anspruch 11).

Bei Fasern, die in einem Ziehverfahren gezogen werden, wie dies beispielsweise bei Lichtleitfasern, aber auch bei Drähten der Fall ist, wird bei dieser Vorrichtung zweckmäßigerweise so vorgegangen, daß bereits während des Ziehvorganges die frisch gezogene Faser durch den Lichtstrahl gezogen und das von dem beleuchteten Abschnitt der Faser erzeugte Raumfrequenzspektrum laufend ausgewertet wird (Anspruch 12).

Ein besonderer Vorteil eines erfindungsgemäßen Verfahrens liegt in seiner Translationsvarianz, die beispielsweise bewirkt, daß eine Bewegung einer zu prüfenden Faser senkrecht zu ihrer Achse das Raumfrequenzspektrum nicht beeinflußt.

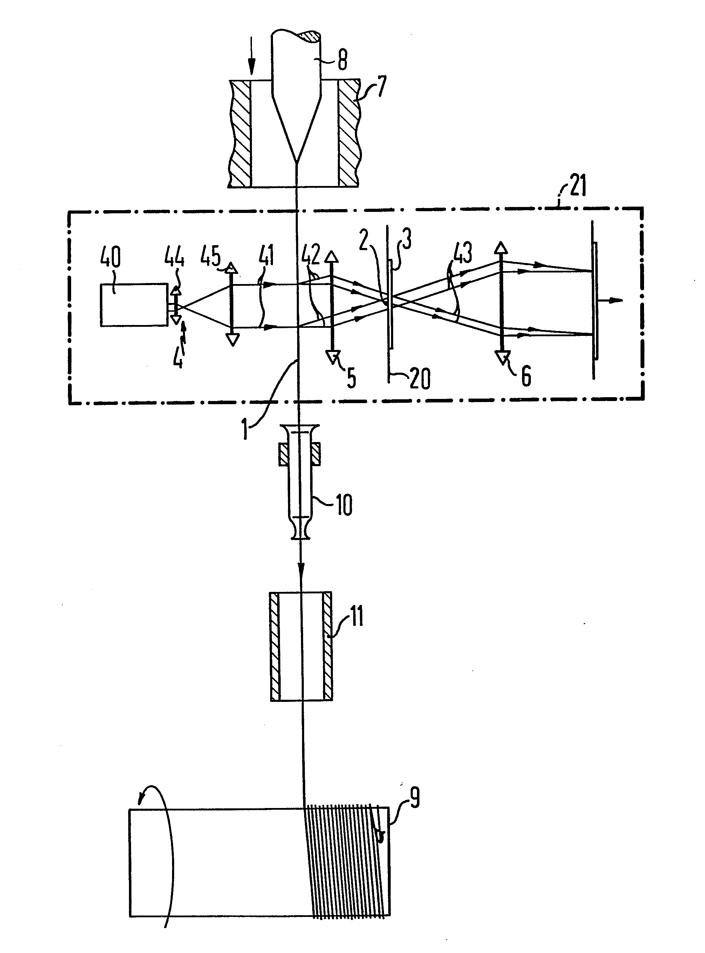

Die Erfindung wird anhand der Figur in der folgenden Beschreibung näher erläutert.

Die Figur zeigt in schematischer Darstellung eine Faserziehvorrichtung, bei der eine Glasfaser aus einem Lichtleitstab gezogen und die frisch gezogene Faser durch eine Vorrichtung zum Prüfen der Faser laufend überprüft wird.

Die in der Figur dargestellte Faserziehvorrichtung umfaßt einen ringförmigen Ziehofen 7, in dessen Öffnung das untere Ende des Lichtleitstabes 8 angeordnet ist.

Der Ziehofen 7 bringt das untere Ende des Lichtleitstabes zum Schmelzen und aus der Schmelzzone wird die
Lichtleitfaser 1 nach unten abgezogen. Dies geschieht
mittels einer Ziehtrommel 9, auf welche die Faser 1
aufgewickelt wird und über die auch die Ziehgeschwindigkeit bestimmt wird.

Bevor die Faser 1 auf die Ziehtrommel 9 aufgewickelt
wird, durchläuft sie noch eine Beschichtungsvorrichtung 10, in welcher sie mit einer Kunststoffschicht
beschichtet wird, und einen Trockenofen 11, in welchem
die noch weiche Kunststoffschicht ausgehärtet wird.

Die Vorrichtung zum Prüfen der Faser 1 umfaßt eine Lichtquelle 4, die einen kohärenten parallelen Lichtstrahl
41 erzeugt, der die Faser 1 senkrecht zur Achse beleuchtet. Das Beleuchtungssystem besteht aus einem
Laser 40 und aus einem aus zwei Sammellinsen 44 und
45 aufgebauten Telesystem, welches als Strahlaufweitungsoptik dient. Im vorliegenden Fall können als
Sammellinsen auch Zylinderlinsen verwendet werden,
weil eine Strahlaufweitung nur in Längsrichtung der
Faser 1 erforderlich ist.

Im Strahlengang 42 des durch die Faser 1 hindurchgegangenen Lichts ist eine Transformationslinse 5 angeordnet, welche in der Brennebene 20, die auf der von
der Lichtquelle abgewandten Seite liegt, das zu dem
durchleuchteten Abschnitt der Faser 1 gehörende Raumfrequenzspektrum erzeugt.

Die Transformationslinse 5 besteht aus einer sphärischen Sammellinse, vorzugsweise einem Objektiv. Sie
könnte aber auch eine zylindrische Sammellinse sein.

In der Brennebene 20 ist das Raumfrequenzfilter 3 angeordnet. Das Raumfrequenzfilter 3 kann beispielsweise aus einer Fotoplatte oder einem Film bestehen, auf die oder den das Raumfrequenzspektrum 2 aufgezeichnet worden ist.

Das Raumfrequenzfilter 3 kann das Raumfrequenzspektrum eines fehlerfreien Faserstücks und damit das Spektrum eines Solleigenschaften aufweisenden Objekts sein.

Ein derartiges Raumfrequenzspektrum kann beispielsweise mit derselben Vorrichtung hergestellt werden, indem in den Strahlengang 41 anstelle der Lichtleitfaser 1 das fehlerfreie Faserstück gebracht wird, und mit dem damit erzeugten Raumfrequenzspektrum eine in der Brennebene 20 angeordnete Fotoplatte belichtet wird. Die entwickelte Fotoplatte bildet dann das Raumfrequenzfilter 3, das allerdings nicht das Positiv des Raumfrequenzspektrums mit Solleigenschaften, sondern dessen Negativ als Schwarz-Weiß-Information enthält. Dies hat aber den Vorteil, daß das Raumfrequenzfilter 3, wenn es mit dem von der Faser 1 erzeugten Raumfrequenzspektrum zur Deckung gebracht worden ist, nur Licht durchläßt, welches Information über Fehler der Fasern oder generell Abweichungen von Solleigenschaften der Faser hindurchläßt, während alles übrige Licht ausgeblendet wird. Auf diese Weise kann die Information über Lage, Größe und Art von Fehlstellen allein ermittelt und ausgewertet werden.

Weist die Faser 1 beispielsweise keine Fehler oder Fehlstellen auf, so entsteht das gleiche Raumfrequenzspektrum wie bei dem fehlerfreien Faserstück, mit dem das Raumfrequenzfilter 3 hergestellt worden ist. Bringt man dieses Raumfrequenzspektrum mit jenem im Negativ dargestellten Raumfrequenzspektrum des Raumfrequenzfilters 3 zur Deckung, derart, daß Intensitätsmaxima und -minima des Raumfrequenzspektrums mit den Schwärzungsmaxima und -minima der Fotoplatte zusammenfallen, so kann wegen der Gleichheit beider Raumfrequenzspektren kein Licht durch die Fotoplatte hindurchgehen. Enthält dagegen die Faser 1 Fehlstellen, so wird an diesen Licht gebeugt oder gestreut, was bedeutet, daß nunmehr Licht an lichtdurchlässige Stellen der Fotoplatte gelangt.

Das durch das Raumfrequenzfilter 3 hindurchgegangene Licht wird von einer Rücktransformationslinse, die ebenfalls aus einer Sammellinse besteht, rücktransformiert. Bei dieser Rücktransformation entsteht in einer Bildebene 21, die der Brennebene 20 zugeordnet ist, ein reelles Bild allein der in dem beleuchteten Faserstück der Faser 1 enthaltenen Fehler oder Fehlstellen. Diese könnten mit verschiedenen Methoden, beispielsweise visuell über eine Fernsehkamera oder über ein lichtempfindliches Detektorfeld und einem Rechner oder einem Kurvenschreiber ausgewertet werden.

Die ständige Auswertung des wiedergegebenen Bildes gibt beispielsweise Aufschluß über Lage, Art und Größe von Fehlstellen, beispielsweise ob es sich um Teilchen, Riefen oder Brechzahlschwankungen handelt. Aber auch Durchmesserschwankungen der Faser können erfaßt werden, so daß das Verfahren auch als Durchmesser-Prüfgerät für Fasern geeignet ist.

Bei Lichtleitfasern, aber auch bei anderen transparenten Fasern, beispielsweise Kunststoffasern, tritt eine Besonderheit insoweit auf, daß als Fehler vielfach Riefen in der Faseroberfläche auftreten, die zudem meist auch noch in Längsrichtung der Faser verlaufen. Derartige Riefen sind längliche Gebilde und somit der Faser selbst in gewisser Weise gestaltähnlich.

Diese Riefen sind Phasenobjekte, welche im wesentlichen nur die Phase und damit die Richtung des durchstrahlenden Lichts ändern. Die etwa in Längsrichtung der Faser verlaufenden Riefen ändern wegen ihrer Gestaltähnlichkeit der Faser die Richtung des Lichts in ähnlicher Weise wie das Phasenobjekt Faser selbst. Das bedeutet, daß die von derartigen Riefen in der Brennebene erzeugten Beugungsmaxima im wesentlichen auf einer zur Faserachse senkrechten Linie in der Brennebene liegen.

Die Beugungsmaxima, die von anderen Faserfehlern erzeugt werden, welche keine den in Faserlängsrichtung ausgerichteten Riefen entsprechende Ordnung aufweisen, beispielsweise Schmutzteilchen oder andere lichtabsorbierende und damit Amplitudenobjekte bildende Fehler oder statistische Brechungsindexschwankungen treten dagegen auch außerhalb der besagten Linie und neben den von der Faser und den Längsriefen erzeugten Beugungsmaxima auf.

Die auf der besagten Linie liegenden und von der Faser und den Längsriefen erzeugten Beugungsmaxima treten im Vergleich zu den von den sonstigen Faserfehlern, insbesondere den Amplitudenobjekten, erzeugten Beugungsmaxima relativ stark in Erscheinung.

Der Intensitäts- oder Helligkeitsunterschied sowohl innerhalb jedes Beugungsmaximums als auch zwischen den Beugungsmaxima kann bei Fasern, insbesondere auch bei fehlerfreien Fasern, so groß sein, daß bei einer fotografischen Aufnahme des Raumfrequenzspektrums zwangsweise eine Über- oder Unterbelichtung des fotografischen Films oder der fotografischen Platte auftritt und dadurch die fotografische Herstellung des Raumfrequenzspektrums zu Schwierigkeiten führen kann.

Diese Schwierigkeiten können dadurch beseitigt werden, daß das Raumfrequenzspektrum des Filters nicht fotografisch, sondern synthetisch mit Hilfe eines Rechners und eines Plotters oder Kurvenschreibers hergestellt wird.

Dieses Verfahren läßt sich nicht nur bei Fasern, sondern überall dort vorteilhaft anwenden, wo hohe Intensitäts- oder Helligkeitsunterschiede bei einer fotografischen Aufnahme des Raumfrequenzspektrums zu Schwierigkeiten führen.

Die bei Fasern in Erscheinung tretende Besonderheit, daß vielfach Längsriefen als Faserfehler auftreten, ermöglicht ein weiteres besonders einfaches, aber wirksames Verfahren zum Prüfen derartiger Fasern.

Da, wie schon erwähnt, die von der Faser selbst und diesen Längsriefen auf der besagten Linie erzeugten Beugungsmaxima im Vergleich zu den von den übrigen Faserfehlern erzeugten Maxima relativ stark sind, treten im rekonstruierten Bild die Faser und diese Längsriefen dominierend in Erscheinung, wenn keine Ortsfrequenzfilterung durchgeführt wird. Meist treten andere Fehler, insbesondere Amplitudenobjekte, kaum noch oder überhaupt nicht mehr in Erscheinung.

Es wurde nun gefunden, daß Faserfehler deutlicher sichtbar gemacht werden können, wenn lediglich die auf der besagten Linie in der Brennebene liegenden und damit von der Faser selbst und den Längsriefen erzeugten Beugungsmaxima wenigstens teilweise ausgeblendet werden.

Dazu reicht aber ein lichtundurchlässiges Gebilde aus, welches in seiner Form der Faser selbst ähnelt, beispielsweise ein Draht oder ein lichtundurchlässiger Streifen, der im Lichtstrahlengang so angeordnet wird, daß alle auf der besagten Linie in der Brennebene liegenden Beugungsmaxima zumindest teilweise ausgeblendet werden.

Über den Grad der Ausblendung dieser Beugungsmaxima kann der Kontrast des rekonstruierten Bildes stufenlos geändert werden. Je mehr die auf der besagten Linie liegenden starken Beugungsmaxima ausgeblendet werden, desto deutlicher treten Amplitudenobjekte in Erscheinung. Je weniger man von diesen starken Maxima ausblendet, desto deutlicher treten Phasenobjekte in Erscheinung. Der Grad der Ausblendung der starken Maxima läßt sich

-12-   VPA   81 P 7 0 6 9 E

über den Durchmesser oder die Breite des Drahtes bzw. Streifens einstellen.

Das soeben geschilderte Verfahren läßt sich vorteilhaft immer dann anwenden, wenn in einem zu prüfenden Objekt ein gewisser Fehler besonders häufig auftritt, dieser Fehler aus einem Phasenobjekt besteht und alle diese Phasenobjekte sich in ihrer Form ähneln und im wesentlichen gleich orientiert sind. Diese geordneten Phasenobjekte können bei der Erzeugung des Raumfrequenzspektrums so zusammenwirken, daß sie zu relativ starken Beugungsmaxima im Raumfrequenzspektrum führen, indem sich die Ordnung der Phasenobjekte wiederspiegelt. Besonders günstig liegen die Verhältnisse zudem noch dann, wenn das zu prüfende Objekt selbst ein Phasenobjekt ist, dem jedes der besagten geordneten Phasenobjekte ihrer Form nach ähnelt und im wesentlichen so orientiert ist, wie das Objekt selbst. Voraussetzung für die Anwendbarkeit dieses Verfahrens ist aber, daß Beugungsmaxima anderer Fehler, insbesondere von Amplitudenobjekten, außerhalb der von den geordneten Phasenobjekten und gegebenenfalls dem Objekt erzeugten Beugungsmaxima vorhanden sind. Unter dieser Voraussetzung kann dann häufig mit einer der Form nach dem Objekt oder einem der geordneten Phasenobjekte ähnlichen Struktur die Raumfrequenzfilterung vorgenommen werden.

Wie früher schon darauf hingewiesen worden ist, kann zum Prüfen des Brechzahlverlaufs und der Faserfestigkeit auch der Lichtleitstab 8 verwendet werden, wobei dann lediglich dieser Stab mit einem kohärenten Lichtstrahl zu beleuchten und von ihm ein Raumfrequenzspektrum zu erzeugen wäre. Es kann dabei jede der drei vorstehend beschriebenen Möglichkeiten der Ortsfrequenzfilterungen verwendet werden, d.h. die Filterung kann mit einem fotografisch erzeugten Raumfrequenzfilter, mit einem synthetisch erzeugten Raumfrequenzfilter oder mit einem quer zur Achse des Stabes verlaufenden länglichen Streifen oder einem ähnlichen Gebilde vorgenommen werden. Wie bei der Faser wird man die für den konkreten Fall am besten geeignete Methode wählen.

Bei der in der Figur dargestellten Vorrichtung schadet eine Verschiebung des zu prüfenden Objekts also der Faser 1, nicht, weil das Verfahren translationsinvariant ist.

Es sei abschließend noch einmal ausdrücklich darauf hingewiesen, daß bei einem hier beschriebenen Verfahren zum Überprüfen von Glasfasern Fehlstellen bereits in einer Vorform oder direkt beim Faser ziehen vor Beschichtung der Faser geortet werden können und ihre Größe abgeschätzt werden kann. Dadurch ist es möglich, Lichtleitfasern für die optische Nachrichtenübertragung zerstörungsfrei zu prüfen, insbesondere auf ihre Zerreißfestigkeit aber auch auf Brechzahlschwankungen, aus denen auf die Übertragungseigenschaften der Fasern geschlossen werden kann. Auch Dickenschwankungen können gemessen werden.

Es sei auch noch einmal ausdrücklich betont, daß das Verfahren nicht auf die Prüfung von Glasfasern beschränkt ist, sondern daß es generell als Verfahren zum zerstörungsfreien Prüfen von Objekten verwendet werden kann.

12 Patentansprüche

1 Figur

Patentansprüche                          VPA 81 P 7069 E

1. Verfahren zum Prüfen von Objekten, insbesondere von Lichtleitfasern oder Vorformen, aus denen Lichtleitfasern hergestellt werden, d a d u r c h   g e - k e n n z e i c h n e t ,  daß von dem Objekt (1) oder einem Teil davon ein Raumfrequenzspektrum (2) erzeugt wird, daß eine Raumfrequenzfilterung vorgenommen wird, und daß der gefilterte Anteil des Spektrums ausgewertet wird.

2. Verfahren nach Anspruch 1, d a d u r c h   g e - k e n n z e i c h n e t ,   daß die Raumfrequenzfilte- rung mit einem Raumfrequenzfilter (3) vorgenommen wird, welches das Raumfrequenzspektrum eines Solleigenschaften aufweisenden oder fehlerfreien Objektes aufweist.

3. Verfahren nach Anspruch 2, d a d u r c h   g e - k e n n z e i c h n e t ,  daß die Raumfrequenzfilte- rung mit einem fotografisch erzeugten Raumfrequenzfilter vorgenommen wird.

4. Verfahren nach Anspruch 2, d a d u r c h   g e - k e n n z e i c h n e t ,   daß die Raumfrequenz- filterung mit einer synthetisch mit Hilfe eines Rech- ners und Kurvenschreibers hergestellten Raumfrequenz- filters vorgenommen wird.

5. Verfahren nach Anspruch 1, d a d u r c h   g e - k e n n z e i c h n e t ,  daß die Filterung mit einem Raumfrequenzspektrum vorgenommen wird, daß aus einer einfachen Struktur besteht, mit welcher nicht alle, sondern nur gewisse, relativ stark in Erscheinung tretende Beugungsmaxima des Raumfrequenzspektrums wenigstens teilweise ausgeblendet werden.

0069355

81 P 7069 E

6. Verfahren nach Anspruch 5 zum Prüfen von transparenten Fasern oder Stäben, d a d u r c h g e - k e n n z e i c h n e t , daß mit einem länglichen lichtundurchlässigen Streifen oder einem ähnlichen länglichen Gebilde die auf einer quer zur Faser- oder Stabachse verlaufenden Linie liegenden Beugungsmaxima des Raumfrequenzspektrums wenigstens teilweise ausgeblendet werden.

7. Verfahren nach einem der vorhergehenden Ansprüche, d a d u r c h g e k e n n z e i c h n e t , daß das durch das Raumfrequenzfilter (3) hindurchgegangene Licht ausgewertet wird.

8. Verfahren nach Anspruch 7, d a d u r c h g e - k e n n z e i c h n e t , daß das in dem durch das Raumfrequenzfilter hindurchgegangene Licht kodiert enthaltene Bild rekonstruiert und ausgewertet wird.

9. Verfahren nach Anspruch 7 oder 8, d a d u r c h g e k e n n z e i c h n e t , daß das durch das Raumfrequenzfilter hindurchgegangene Licht oder das rekonstruierte Bild visuell über eine Fernsehkamera oder mittels eines Detektorfeldes und eines Kurvenschreibers oder Rechners ausgewertet wird.

10. Verfahren nach Anspruch 7, d a d u r c h g e - k e n n z e i c h n e t , daß die Intensität des durch das Raumfrequenzfilter hindurchgegangenen Lichts abgetastet und ausgewertet wird.

11. Vorrichtung zur Durchführung eines Verfahrens nach einem der vorhergehenden Ansprüche, g e k e n n - z e i c h n e t d u r c h ein einen kohärenten Lichtstrahl (41) erzeugendes Beleuchtungssystem (4) und durch eine auf der Strahlachse angeordnete Transformationslinse (5), zwischen der und dem Beleuchtungssystem (4) das zu prüfende Objekt (1) im Strahl (41) anzuordnen ist und in einer deren Brennebenen das Raumfrequenzfilter (3) anzuordnen ist.

12. Verfahren zum Prüfen einer in einem Ziehverfahren gezogenen Faser mit einer Vorrichtung nach Anspruch 11, d a d u r c h  g e k e n n z e i c h n e t , daß bereits während des Ziehvorganges die frisch gezogene Faser (1) durch den Lichtstrahl (41) gezogen und das von dem beleuchteten Abschnitt der Faser erzeugte Raumfrequenzspektrum ständig ausgewertet wird.